(19) Europäisches Patentamt
European Patent Office
Office européen des brevets

(11) Veröffentlichungsnummer: **0 229 641**
**B1**

(12) # EUROPÄISCHE PATENTSCHRIFT

(45) Veröffentlichungstag der Patentschrift: **03.10.90**

(51) Int. Cl.$^5$: **C 07 C 249/12**

(21) Anmeldenummer: **87100171.5**

(22) Anmeldetag: **09.01.87**

(54) **Verfahren zur Herstellung von Hydroxybenzaldoxim-O-ethern.**

(30) Priorität: **16.01.86 DE 3601036**

(43) Veröffentlichungstag der Anmeldung:
**22.07.87 Patentblatt 87/30**

(45) Bekanntmachung des Hinweises auf die Patenterteilung:
**03.10.90 Patentblatt 90/40**

(84) Benannte Vertragsstaaten:
**BE CH DE FR GB IT LI NL**

(56) Entgegenhaltungen:

**HOUBEN-WEYL: "Methoden der organischen Chemie", Band VI/1c, Teil 1, 1976, Seiten 247-249, Georg Thieme Verlag, Stuttgart, DE;**

**SYNTHESIS, Nr. 3, März 1984, Seite 266, Georg Thieme Verlag, Stuttgart, DE; L. BONSIGNORE et al.: "Novel syntheses with carbon suboxide; V. Cyclocondensation with 2-(arylimino)-methylrbenzenamines or 2-aminobenzaldehyde oximes to form 2-oxo-1,2-dihydroquinoline derivatives"**

(73) Patentinhaber: **BAYER AG**
**D-5090 Leverkusen 1 Bayerwerk (DE)**

(72) Erfinder: **Elbe, Hans-Ludwig, Dr.**
**DasnÖckel 59**
**D-5600 Wuppertal 11 (DE)**
Erfinder: **Lantzsch, Reinhard Dr.**
**Am Buschhäuschen 51**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 229 641 B1**

**Beschreibung**

Die Erfindung betrifft ein neues Verfahren zur Herstellung von bekannten Hydroxybenzaldoxim-O-ethern, die als Zwischenprodukte zur Synthese von Verbindungen mit fungizider, insektizider und antimykotischer Wirksamkeit verwendet werden können.

Es ist bereits bekannt geworden, daß man bestimmte Hydroxy-benzaldoxim-O-ether herstellen kann, indem man Hydroxybenzaldehyde mit den entsprechenden Hydroxylamin-Derivativen umsetzt (vgl. EP-OS 0 076 370 und EP-OS 0 115 828). Die betreffende Umsetzung läßt sich durch das folgende Reaktionsschema veranschaulichen:

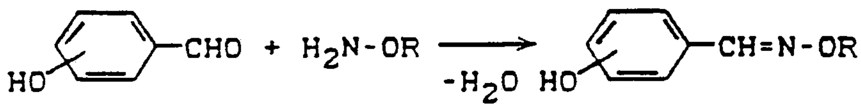


R = Alkyl, Alkenyl, Alkinyl.

Nachteilig an diesem Verfahren ist jedoch, daß die Ausgangsstoffe nur durch aufwendige Synthese zugänglich sind. Sie sind deshalb relativ kostspielig und ihr Einsatz für eine Herstellung von Hydroxy-benzaldoxim-O-ethern in technischem Maßstab ist problematisch. Außerdem sind die Ausbeuten an Hydroxy-benzaldoxim-O-ethern bei dem oben angegebenen Verfahren nicht immer befriedigend.

Es wurde nun gefunden, daß man bekannte Hydroxy-benzaldoxim-O-ether der Formel

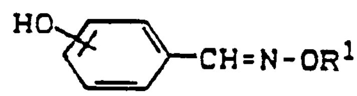 (I)

in welcher

$R^1$ für Alkyl, Alkenyl oder Alkinyl steht,

erhält, wenn man Aminobenzaldoxim-O-ether der Formel

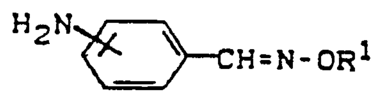 (II)

in welcher

$R^1$ die oben angegebene Bedeutung hat,

mit einem Diazotierungsmittel in saurer, wäßriger Lösung umsetzt und die entstehenden Diazoniumsalze der Formel

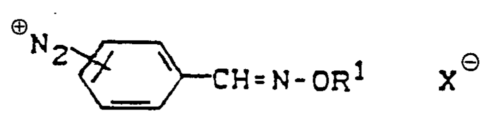 (III)

in welcher

$X^{\ominus}$ für ein Äquivalent eines anorganischen Anions steht und

$R^1$ die oben angegebene Bedeutung hat,

ohne Zwischenisolierung in saurer, wäßriger Lösung thermisch hydrolysiert.

Es ist als äüßerst überraschend zu bezeichnen, daß die erfindungsgemäße Umsetzung unter den angegebenen Verfahrensbedingungen glatt und in guter Ausbeute abläuft. Es war nämlich nicht zu erwarten, daß das bei der thermischen Hydrolyse des Diazoniumsalzes auftretende reaktive Carbo-Kation (am Aromaten) selektiv in der gewünschten Art und Weise reagiert und kein intermolekularer Angriff am freien Elektronenpaar des Oximether-Stickstoffs erfolgt.

Das erfindungsgemäße Verfahren zeichnet sich durch eine Reihe von Vorteilen aus. So ermöglicht es die Herstellung von Hydroxy-benzaldoxim-O-ethern der Formel (I) in hohen Ausbeuten, wobei billige und gut zugängliche Verbindungen als Ausgangsprodukte eingesetzt werden. Ferner ist die Umsetzung einfach durchführbar und die Isolierung der Benzaldoxim-O-ether der Formel (I) bereitet keinerlei Schwierigkeiten. Das erfindungsgemäße Verfahren ist daher besonders geeignet, um Hydroxy-benzaldoxim-O-ether der Formel (I) in technischem Maßstab herzustellen.

Verwendet man beispielsweise 4-Amino-benzaldoxim-O-ethyl-ether als Ausgangstoff, wäßrige Schwefelsäure als saures Medium und Natriumnitrit als Diazotierungsmittel, so kann der Verlauf des erfindungsgemäßen Verfahrens durch das folgende Formelschema veranschaulicht werden:

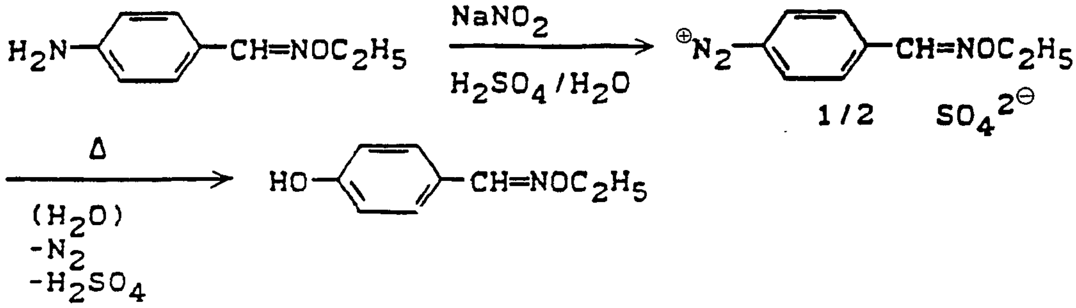


Die bei dem erfindungsgemäßen Verfahren als Ausgangsstoffe zur verwendenden Amino-benzaldoxim-O-ether sind durch die Formel (II) allgemein definiert. In dieser Formel steht $R^1$ vorzugsweise für geradkettiges oder verzweigtes Alkyl mit 1 bis 20 Kohlenstoffatomen sowie für jeweils geradkettiges oder verzweigtes Alkenyl und Alkinyl mit jeweils 3 bis 20 Kohlenstoffatomen.

Bevorzugt sind dabei jeweils die 4-Amino-benzaldoxim-O-ether.

Besonders bevorzugte Ausgangsstoffe sind diejenigen Amino-benzaldoxim-O-ether der Formel (II), in denen $R^1$ für geradkettiges oder verzweigtes Alkyl mit 1 bis 10 Kohlenstoffatomen sowie für jeweils geradkettiges oder verzweigtes Alkenyl und Alkinyl mit jeweils 3 bis 10 Kohlenstoffatomen steht.

Besonders bevorzugt sind dabei jeweils die 4-Amino-benzaldoxim-O-ether.

Ganz besonders bevorzugte Ausgangsstoffe sind diejenigen 4-Amino-benzaldoxim-O-ether der Formel (II), in denen $R^1$ für geradkettiges oder verzweigtes Alkyl mit 1 bis 4 Kohlenstoffatomen steht.

Die Amino-benzaldoxim-O-ether Formel (II) sind teilweise bekannt. So wird in Synthesis *1984*, 266, der 2-Amino-benzald-oxim-O-methylether beschrieben.

Neu sind die Amino-benzaldoxim-O-ether der Formel

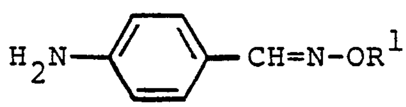

(IIa)

in welcher

$R^1$ für geradkettiges oder verzweigtes Alkyl mit bis 20 Kohlenstoffatomen, Alkenyl mit 3 bis 20 Kohlenstoffatomen oder Alkinyl mit 3 bis 20 Kohlenstoffatomen steht.

Die Amino-benzaldoxim-O-ether der Formel (II) lassen sich herstellen, indem man Amino-benzaldehyde der Formel

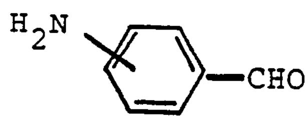

(IV)

mit Hydroxylamin-O-ethern der Formel

$$H_2N—OR^1 \qquad (V)$$

in welcher

$R^1$ die oben angegebene Bedeutung hat,

bzw. deren Halogenwasserstoffsalzen in Gegenwart eines polaren, protischen Verdünnungsmittels, wie zum Beispiel Methanol, Ethanol, n- und i-Propanol, n- und i-Butanol, Wasser oder Eisessig, und gegebenenfalls in Gegenwart einer Base, wie zum Beispiel Natriumhydroxid, Kaliumhydroxid oder Natriumacetat, bei Temperaturen zwischen 0°C und 50°C, vorzugsweise zwischen 10°C und 30°C, umsetzt.

Bei der Durchführung dieses Verfahrens setzt man vorzugsweise auf 1 Mol Amino-benzaldehyd der Formel (IV) 1 bis 4 Mol, vorzugsweise 1 bis 2 Mol Hydroxylamin-O-ether der Formel (V) bzw. deren Halogenwasserstoff-Salze und gegebenenfalls 1 bis 4 Mol, vorzugsweise 1 bis 2 Mol Base ein. Die Reaktionszeit beträgt im allgemeinen 5 bis 15, vorzugsweise 5 bis 10 Stunden. Die Isolierung der Aminobenzaldoxim-O-ether der Formel (II) erfolgt in üblicher Weise, wie z.B. durch fraktionierte Destillation.

Die Amino-benzaldehyde der Formel (IV) sind allgemein bekannte Verbindungen der organischen Chemie (vgl. Beilsteins Handbuch der Organischen Chemie, Band E III 14, S. 47, 53 und 57) bzw. können sie nach den dort angegebenen Verfahren erhalten werden.

Besonders bevorzugt ist der 4-Amino-benzaldehyd.

Die Hydroxylamino-O-ether der Formel (V) sind ebenfalls allgemein bekannte Verbindungen der organischen Chemie (vgl. z.B. Houben-Weyl, Methoden der Organischen Chemie, Band 10/1, S. 1181ff) bzw. können sie nach den dort angegebenen Verfahren erhalten werden.

Als Diazotierungsmittel kommen für das erfindungsgemäße Verfahren alle üblicherweise für eine Diazotierung geeigneten Komponenten infrage. Hierzu gehören vorzugsweise Natriumnitrit, Kaliumnitrit, Ammoniumnitrit sowie Nitrosylschwefelsäure.

Das erfindungsgemäße Verfahren wird in saurer, wäßriger Lösung durchgeführt. Als Säuren kommen verzugsweise anorganische Säuren infrage, wie insbesondere die Schwefel säure.

Dabei haben die Lösungen im allgemeinen eine Konzentration von 1 bis 50 Gewichts%, vorzugsweise 1 bis 30 Gewichts% an Säure.

Die Temperaturen können bei der Durchführung des erfindungsgemäßen Verfahrens in einem größeren Bereich variiert werden.

Bei der Diazotierung arbeitet man allgemein bei Temperaturen zwischen 0 und 20°C, vorzugsweise zwischen 0 und 10°C.

Die anschließende thermische Hydrolyse der Diazoniumsalze wird im allgemeinen bei Temperaturen zwischen 50 und 100°C, vorzugsweise zwischen 70 und 100°C durchgeführt.

Bei der Durchführung des erfindungsgemäßen Verfahrens setzt man auf 1 Mol Aminobenzaldoxim-O-ether der Formel (II) im allgemeinen 1 bis 1,5 Mol, vorzugsweise 1 bis 1,2 Mol Diazotierungsmittel ein. Das überschüssige Diazotierungsmittel kann in üblicher Weise von der thermischen Hydrolyse durch Zugabe von Harnstoff zerstört werden.

In einer bevorzugten Durchführung der erfindungsgemäßen Verfahrens wird die Diazoniumsalzlösung zur thermischen Hydrolyse in eine 50 bis 100°C, vorzugsweise 70 bis 100°C heiße wäßrige Schwefelsäure eindosiert, wobei die Konzentration der Schwefelsäure 1 bis 50 Gewichts%, vorzugsweise 1 bis 30 Gewichts% beträgt. Die Eindosier-Geschwindigkeit wird dabei so gewählt, daß die Temperatur der vorgelegten wäßrigen Schwefelsäure konstant gehalten werden kann. Die Isolierung der Hydroxy-benzaldoxim-O-ether der formel (I) erfolgt in allgemein bekannter Art und Weise (vgl. auch die Herstellungsbeispiele).

Die nach dem erfindungsgemäßen Verfahren herstellbaren Hydroxy-benzaldoxim-O-ether der Formel (I) sind allgemein wertvolle Ausgangstoffe zur Synthese von biologisch aktiven Verbindungen. So lassen sie sich z.B. verwenden zur Synthese von Oximethern, welche gute insektizide Eigenschaften besitzen (vgl. EP-OS 0 115 828); von Azolylphenoxy-Derivaten, welche hervorragende fungizide Eigenschaften aufweisen (vgl. EP-OS 0 076 370); von 1-Hydroxyethyl-triazolyl-Derivaten, welche gute fungizide und antimykotische Eigenschaften aufweisen (vgl. EP-OS 0 110 048 und DE-OS 3 314 548); sowie von Hydroxyalkylazolyl-Derivaten, welche gute antimykotische Wirksamkeit besitzen (vgl. DE-OS 3 427 844).

So läßt sich beispeilsweise das 3,3-Dimethyl-1-(4-methoximinomethyl-phenoxy)-1-(1,2,4-triazol-1-yl)-butan-2-on der Formel

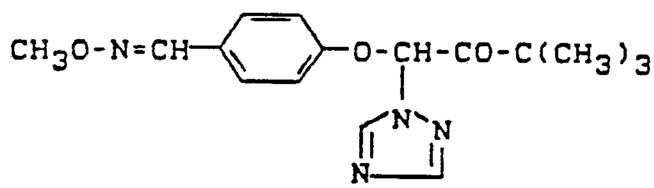

herstellen, indem man 3,3-Dimethyl-1-(1,2,4-triazol-1-yl)-butan-2-on zunächst mit Brom zum 1-Brom-(1,2,4-triazol-1-yl)-3,3-dimethyl-butan-2-on umsetzt und dieses anschließend mit 4-Hydroxybenzaldehyd-O-methyl-oximether in Gegenwart einer Base umsetzt. Diese Synthese läßt sich formelmäßig wie folgt veranschaulichen:

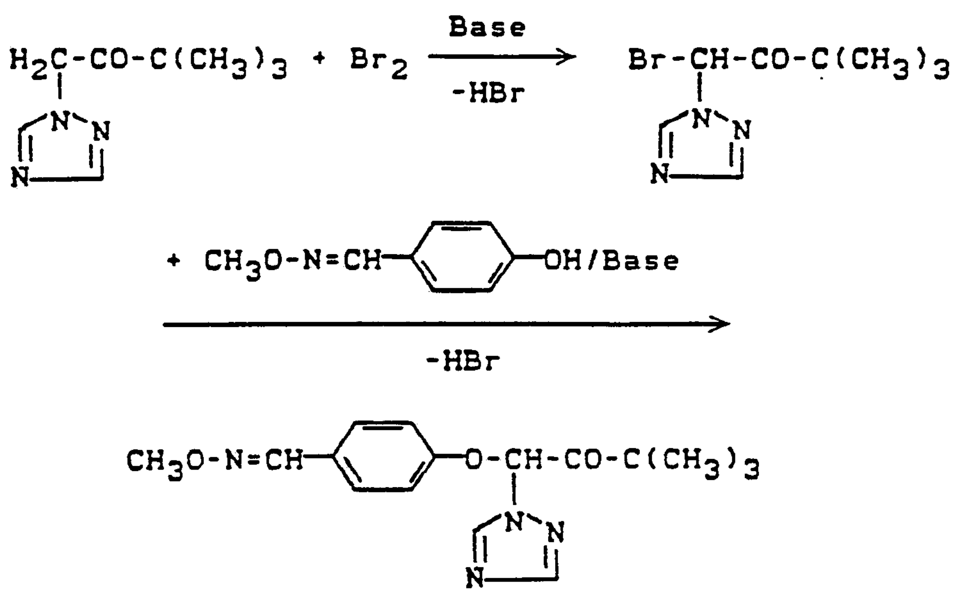


Das erfindungsgemäße Verfahren wird durch das nachfolgende Beispiel veranschaulicht.

Beispiel 1

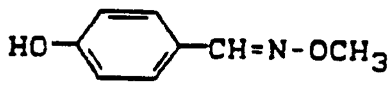

(I-1)

30 g (0,91 Mol) 4-Methoxyiminomethylanilin (Gehalt: 95,5%) werden in 120 g 25%-iger wäßriger Schwefelsäure aufgeschlämmt. Man läßt 30 Minuten bei Raumtemperatur nachrühren und kühlt sodann auf 5—10°C ab.

Innerhalb von 30 Minuten läßt man eine Lösung von 13,8 g (0,2 Mol) Natriumnitrit in 80 ml Wasser in das Reaktionsgemisch eintropfen.

Man rührt 1 Stunde bei 5—10°C nach und zerstört anschließend überschüssiges Nitrit durch zugesetzten Harnstoff.

Zum Hydrolisieren läßt man die Diazoniumlösung möglichst rasch in vorgelegte 80—85°C heiße, wäßrige Schwefelsäure (120 g, 25%-ig) einlaufen, wobei die Temperatur konstant gehalten wird. Nach Beendigung der Zugabe läßt man 15 Minuten bei 80—85°C nachrühren. Zur Aufarbeitung wird auf Raumtemperatur abgekühlt, mit wäßriger Natronlauge auf pH 5—6 gestellt und mit Methylenchlorid extrahiert. Die organische Phase wird unter vermindertem Druck eingeengt. Das verbleibende Rohprodukt wird im Vakuum destilliert. Man erhält 25,7 g (Gehalt nach GC: 95,4%; Ausbeute: 85% der Theorie) 4-Methoxyiminomethylphenol vom Siedepunkt 140—142°C/0,13 mbar.

Herstellung des Ausgangsproduktes:

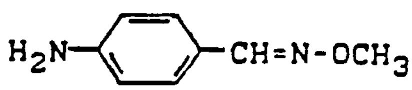

(II-1)

170 g (0,98 Mol) 4-Aminobenzaldehyd (feucht, 70%ig) werden in 1,5 l Methanol gelöst. Man versetzt mit 300 ml Wasser und gibt 119,1 g (1,44 Mol) Natriumacetat sowie 120 g (1,44 Mol) O-Methylhydroxylamin hinzu. Man läßt 10 Stunden bei Raumtemperatur rühren, saugt von anorganischem Rückstand ab und engt das Filterat unter vermindertem Druck ein. Der Rückstand wird unter vermindertem Druck destilliert. Man erhält 134,9 g (Gehalt nach GC: 95,5%; Ausbeute 88% der Theorie) 4-Methoxyiminomethylanilin vom Siedepunkt 110—115°C/0,27 mbar.

Herstellung der Verbindung der Formel:

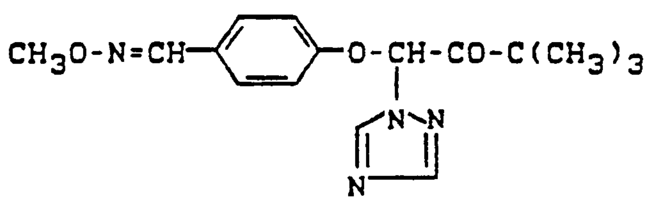

In eine Lösung von 217 g (1,3 Mol) 3,3-Dimethyl-1-(1,2,4-triazol-1-yl)-2-butanon in 700 ml Eisessig trägt man 110 g (1,34 Mol) Natriumacetat ein, wobei die Temperatur auf ca. 28°C ansteigt. Man läßt 30 Minuten nachrühren und versetzt tropfenweise unter leichter Kühlung bei 30 bis 33°C mit 208 g (1,3 Mol) Brom. Das Reaktionsgemisch wird 2,5 Stunden bei Raumtemperatur nachgerührt und in 1200 ml Wasser gegeben. Man extrahiert mit Methylenchlorid, wächst mit Wasser und wässriger Bicarbonatlösung, trocknet über Natriumsulfat und engt ein.

Das so erhaltene rohe 1-Brom-3,3-Dimethyl-1-(1,2,4-triazol-1-yl)-2-butanon wird in 100 ml Acetonitril gelöst und zu einer Aufschlämmung von 151 g (1 Mol) 4-Methoximinomethylphenol und 150 g (1,09 Mol) Kaliumcarbonat in 800 ml Acetonitril gegeben, wobei die Temperatur auf etwa 40°C ansteigt. Man läßt das Reaktionsgemisch 3 Stunden bei 60 bis 65°C nachrühren, kühlt anschließend ab und gibt auf Wasser. Man extrahiert mit Toluol, wäscht mit Wasser, trocknet und engt ein. Der Rückstand wird in Ligroin verrieben und auf Ton getrocknet. Man erhält 241 g (76% der Theorie) 3,3-Dimethyl-1-(4-methoxyiminomethyl-phenoxy)-1-(1,2,4-triazol-1-yl)-butan-2-on vom Schmelzpunkt 83—87°C.

**Patentansprüche**

1. Verfahren zur Herstellung von Hydroxy-benzaldoxim-O-ethern der Formel

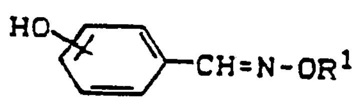

(I)

in welcher

$R^1$ für Alkyl, Alkenyl oder Alkinyl steht,
dadurch gekennzeichnet, daß man Aminobenzaldoxim-O-ether der Formel

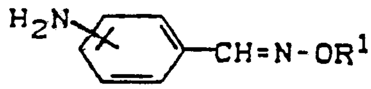

(II)

in welcher
$R^1$ die oben angegebene Bedeutung hat,
mit einem Diazotierungsmittel in saurer, wäßriger Lösung umsetzt und die entstehenden Diazoniumsalze der Formel

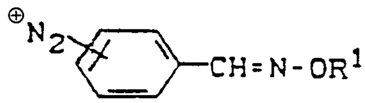

$X^{\ominus}$ (III)

in welcher
$X^{\ominus}$ für ein Äquivalent eines anorganischen Anions steht und
$R^1$ die oben angegebene Bedeutung hat,
ohne Zwischenisolierung in saurer, wäßriger Lösung thermisch hydrolysiert.

2. Verfahren gemäß Anspruch 1, dadurch gekennzeichnet, daß man Amino-benzoaldoxim-O-ether der Formel (II) einsetzt, in denen $R^1$ für geradkettiges oder verzweigtes Alkyl mit 1 bis 20 Kohlenstoffatomen, Alkenyl mit 3 bis 20 Kohlenstoffatomen oder Alkinyl mit 3 bis 20 Kohlenstoffatomen steht.

3. Verfahren gemäß Anspruch 1, dadurch gekennzeichnet, daß man Amino-benzaldoxim-O-ether der Formel (II) einsetzt, in denen $R^1$ für geradkettiges oder verzweigtes Alkyl mit 1 bis 10 Kohlenstoffatomen, Alkenyl mit 3 bis 10 Kohlenstoffatomen oder Alkinyl mit 3 bis 10 Kohlenstoffatomen steht.

4. Verfahren gemäß Anspruch 1, dadurch gekennzeichnet, daß man als Ausgangsstoffe der Formel (II) 4-Aminobenzaldoxim-O-ether einsetzt, in denen $R^1$ für geradkettiges oder verzweigtes Alkyl mit 1 bis 4 Kohlenstoffatomen steht.

5. Verfahren gemäß Anspruch 1, dadurch gekennzeichnet, daß man Natriumnitrit, Kaliumnitrit, Ammoniumnitrit oder Nitrosylschwefelsäure als Diazotierungsmittel einsetzt.

6. Verfahren gemäß Anspruch 1, dadurch gekennzeichnet, daß man wäßrige Schwefelsäure als Acidifizierungsmittel einsetzt.

7. Verfahren gemäß Anspruch 1, dadurch gekennzeichnet, daß man die Diazotierung bei Temperaturen zwischen 0 und 20°C durchführt.

8. Verfahren gemäß Anspruch 1, dadurch gekennzeichnet, daß man die thermische Hydrolyse bei Temperaturen zwischen 50 und 100°C durchführt.

9. Amino-benzaldoxim-O-ether der Formel

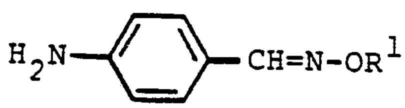

(II)

in welcher
$R^1$ für geradkettiges oder verzweigtes Alkyl mit 1 bis 20 Kohlenstoffatomen, Alkenyl mit 3 bis 20 Kohlenstoffatomen oder Alkinyl mit 3 bis 20 Kohlenstoffatomen steht.

## Revendications

1. Procédé de préparation d'O-éthers d'hydroxybenzaldoximes de formule

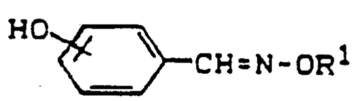

(I)

dans laquelle $R^1$ représente un groupe alkyle, alcényle ou alcynyle, caractérisé en ce que l'on fait réagir des O-éthers d'aminobenzaldoximes de formule

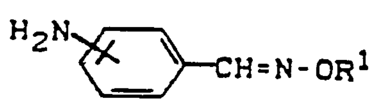

(II)

dans laquelle $R^1$ a les significations indiquées ci-dessus, avec un agent diazotant en solution aqueuse acide, ce qui donne les sels de diazonium de formule

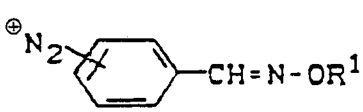

$X^{\ominus}$ (III)

dans laquelle

$X^{\ominus}$ représente un équivalent d'un anion minéral, et

$R^1$ a les significations indiquées ci-dessus,

qu'on hydrolyse à chaud en solution aqueuse acide sans les isoler.

2. Procédé selon la revendication 1, caractérisé en ce que l'on part d'O-éthers d'aminobenzaldoximes de formule II dans laquelle $R^1$ représente un groupe alkyle à chaîne droite ou ramifiée en $C_1$—$C_{20}$, un groupe alcényle en $C_3$—$C_{20}$ ou un groupe alcynyle en $C_3$—$C_{20}$.

3. Procédé selon la revendication 1, caractérisé en ce que l'on part d'O-éthers d'aminobenzaldoximes de formule II dans laquelle $R^1$ représente un groupe alkyle à chaîne droite ou ramifiée en $C_1$—$C_{10}$, un groupe alcényle en $C_3$—$C_{10}$ ou un groupe alcynyle en $C_3$—$C_{10}$.

4. Procédé selon la revendication 1, caractérisé en ce que l'on utilise en tant que composants de départ de formule II des O-éthers de la 4-aminobenzaldoxime pour lesquels $R^1$ représente un groupe alkyle à chaîne droite ou ramifiée en $C_1$—$C_4$.

5. Procédé selon la revendication 1, caractérisé en ce que l'on utilise en tant qu'agent diazotant le nitrate de sodium, le nitrite de potassium, le nitrite d'ammonium ou l'acide nitrosylsulfurique.

6. Procédé selon la revendication 1, caractérisé en ce que l'on utilise en tant qu'agent acidifiant l'acide sulfurique aqueux.

7. Procédé selon la revendication 1, caractérisé en ce que la diazotation est effectuée à des températures de 0 à 20°C.

8. Procédé selon la revendication 1, caractérisé en ce que l'hydrolyse à la chaleur est effectuée à des températures de 50 à 100°C.

9. O-éthers d'aminobenzaldoximes de formule

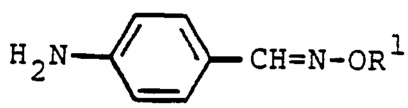 (II)

dans laquelle $R^1$ représente un groupe alkyle à chaîne droite ou ramifiée en $C_1$—$C_{20}$, alcényle en $C_3$—$C_{20}$ ou alcynyle en $C_3$—$C_{20}$.

**Claims**

1. Process for the preparation of hydroxy-benzaldoxime O-ethers of the formula

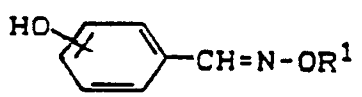 (I)

in which

$R^1$ represents alkyl, alkenyl or alkinyl, characterized in that aminobenzaldoxime O-ethers of the formula

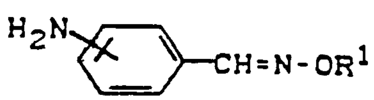 (II)

in which

$R^1$ has the abovementioned meaning, are reacted with a diazotizing agent in acidic, aqueous solution, and the resulting diazonium salts of the formula

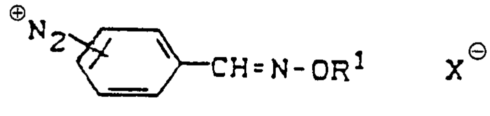 (III)

in which

$X^{\ominus}$ represents one equivalent of an inorganic anion and

$R^1$ has the abovementioned meaning, are thermally hydrolyzed in acidic, aqueous solution without intermediate isolation.

2. Process according to Claim 1, characterized in that amino-benzaldoxime O-ethers of the formula (II) are employed in which $R^1$ represents straight-chain or branched alkyl having 1 to 20 carbon atoms, alkenyl having 3 to 20 carbon atoms or alkinyl having 3 to 20 carbon atoms.

3. Process according to Claim 1, characterized in that amino-benzaldoxime O-ethers of the formula (II) are employed in which $R^1$ represents straight-chain or branched alkyl having 1 to 10 carbon atoms, alkenyl having 3 to 10 carbon atoms or alkinyl having 3 to 10 carbon atoms.

4. Process according to Claim 1, characterized in that 4-amino-benzaldoxime O-ethers in which $R^1$ represents straight-chain or branched alkyl having 1 to 4 carbon atoms are employed as starting materials of the formula (II).

5. Process according to Claim 1, characterized in that sodium nitrite, potassium nitrite, ammonium nitrite or nitrosylsulphuric acid are employed as diazotizing agent.

6. Process according to Claim 1, characterized in that aqueous sulphuric acid is employed as acidifying agent.

7. Process according to Claim 1, characterized in that the diazotization is carried out at temperatures between 0 and 20°C.

8. Process according to Claim 1, characterized in that the thermal hydrolysis is carried out at temperatures between 50 and 100°C.

9. Amino-benzaldoxime O-ethers of the formula

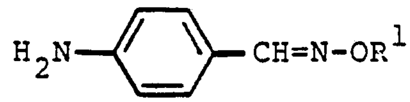

(II)

in which $R^1$ represents straight-chain or branched alkyl having 1 to 20 carbon atoms, alkenyl having 3 to 20 carbon atoms or alkinyl having 3 to 20 carbon atoms.